# EUROPÄISCHE PATENTANMELDUNG

(11) **EP 4 641 024 A1**
(43) Veröffentlichungstag der Anmeldung: **29.10.2025**
(21) Anmeldenummer: 24171717.2
(22) Anmeldetag: 22.04.2024
(51) Int. Cl.: F04D 29/44, F04D 13/06, A61M 60/232

(54) **PUMPENEINHEIT FÜR EINE ZENTRIFUGALPUMPE SOWIE ZENTRIFUGALPUMPE**

(71) Anmelder: Levitronix GmbH, 8048 Zürich (CH)
(72) Erfinder: Steinert, Daniel, Dr., 8180 Bülach (CH); Hu, Rennan, 8057 Zürich (CH); Schmid, Alexander, 8915 Hausen am Albis (CH); Barletta, Natale Dr., 8048 Zürich (CH)
(74) Vertreter: IPS Irsch AG

(57) **Zusammenfassung**

Es wird eine Pumpeneinheit für eine Zentrifugalpumpe vorgeschlagen, welche die Pumpeneinheit und einen Stator (100) umfasst, der sich in einer axialen Richtung (A) von einem ersten axialen Ende (110) bis zu einem zweiten axialen Ende (120) erstreckt, wobei an dem ersten axialen Ende (110) eine becherförmige Ausnehmung vorgesehen ist, in welche die Pumpeneinheit (1) einsetzbar ist, wobei die Pumpeneinheit (1) ein Pumpengehäuse (2) mit einem Einlass (21) und mit einem Auslass (22) für ein zu förderndes Fluid aufweist, sowie einen in dem Pumpengehäuse (2) angeordneten Rotor (10) mit einer Mehrzahl von Flügeln (103) zum Fördern des Fluids, wobei das Pumpengehäuse (2) einen Pumpenraum (23) begrenzt, wobei der Rotor (10) um die axiale Richtung (A) rotierbar ist, wobei die Pumpeneinheit (1) für eine berührungslos magnetische Lagerung des Rotors (10) und für einen berührungslos magnetischen Antrieb des Rotors (10) durch den Stator (100) ausgestaltet ist, wobei das Pumpengehäuse (2) ein Deckelteil (4) und ein Bodenteil (3) aufweist, wobei das Bodenteil (3) einen zylindrischen Becher (31) zur Aufnahme des Rotors (10) aufweist, welcher Becher in die becherförmige Ausnehmung des Stators (100) einsetzbar ist, und wobei der Auslass (22) eine Eintrittsfläche (221) aufweist, durch welche das Fluid aus dem Pumpenraum (23) in den Auslass (22) strömen kann. Die Eintrittsfläche (221) des Auslasses (22) weist ein Profil auf, welches verschieden von einer Kreisfläche ist. Ferner wird eine Zentrifugalpumpe zum Fördern eines Fluids vorgeschlagen, die eine solche Pumpeneinheit (1) umfasst.

## Beschreibung

Die Erfindung betrifft eine Pumpeneinheit für eine Zentrifugalpumpe gemäss dem Oberbegriff des unabhängigen Patentanspruchs. Die Erfindung betrifft ferner eine Zentrifugalpumpe mit einer solchen Pumpeneinheit.

Es sind Zentrifugalpumpen bekannt, welche eine Pumpeneinheit und einen Stator umfassen, der als Antriebseinheit für den Rotor der Pumpeneinheit ausgestaltet ist, wobei der Rotor der Pumpeneinheit das Flügelrad der Zentrifugalpumpe bildet. Dabei ist der Rotor in der Pumpeneinheit mittels des Stators berührungslos magnetisch lagerbar und berührungslos zur Rotation um eine axiale Richtung antreibbar. Solche Zentrifugalpumpen werden beispielsweise von der Anmelderin unter der Produktbezeichnung Levitronix^{®} BPS Pumpen vertrieben.

Der Stator und der Rotor bilden einen elektromagnetischen Drehantrieb. Bei den Levitronix^{®} BPS Pumpen, beispielsweise, ist der elektromagnetische Drehantrieb nach dem Prinzip des lagerlosen Motors ausgestaltet. Mit dem Begriff lagerloser Motor ist dabei ein elektromagnetischer Drehantrieb gemeint, bei welchem der Rotor vollkommen magnetisch bezüglich des Stators lagerbar ist, wobei keine separaten magnetischen Lager vorgesehen sind. Der Stator ist dazu als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation um eine durch die axiale Richtung definierte Solldrehachse bewirkt, und welches zum anderen eine beliebig einstellbare Querkraft auf den Rotor ausübt, sodass dessen radiale Position aktiv steuerbar bzw. regelbar ist. Somit sind drei Freiheitsgrade des Rotors aktiv regelbar, nämlich seine Rotation sowie seine radiale Position (zwei Freiheitsgrade). Bezüglich dreier weiterer Freiheitsgrade, nämlich seiner Position in axialer Richtung und Verkippungen bezüglich der zur Solldrehachse senkrechten radialen Ebene (zwei Freiheitsgrade), ist der Rotor passiv magnetisch, das heisst nicht ansteuerbar, durch Reluktanzkräfte gelagert bzw. stabilisiert. Das Nichtvorhandensein eines separaten magnetischen Lagers bei vollständiger magnetischer Lagerung des Rotors ist die Eigenschaft, welcher der lagerlose Motor seinen Namen verdankt. In dem Lager- und Antriebsstator lässt sich die Lagerfunktion nicht von der Antriebsfunktion separieren.

Es sind natürlich auch andere Ausgestaltungen von Zentrifugalpumpen bekannt, bei welchen der Rotor berührungslos magnetisch gelagert ist, beispielsweise solche bei denen separate Magnetlager für den Rotor vorgesehen sind, sodass die magnetische Lagerfunktion von der Antriebsfunktion getrennt ist. Beispielsweise sind dazu separate Spulen vorgesehen, mit denen nur die Lagerkräfte für den Rotor realisiert werden, die aber keinen Beitrag zum Antrieb des Rotors leisten. Eine solche Zentrifugalpumpe ist beispielsweise in der WO 2022/004144 offenbart.

Zentrifugalpumpen mit berührungslos magnetisch gelagerten und angetriebenen Rotoren, beispielsweise solche, die nach dem Prinzip des lagerlosen Motors ausgestaltet und betrieben werden, haben sich in einer Vielzahl von Anwendungen bewährt. Aufgrund der Abwesenheit von mechanischen Lagern eignen sich solche Zentrifugalpumpen für Anwendungen, bei denen sehr empfindliche Substanzen gefördert werden, beispielsweise Blutpumpen, oder bei denen sehr hohe Anforderungen an die Reinheit gestellt werden, beispielsweise in der Halbleiterindustrie, der pharmazeutischen Industrie, der biotechnologischen Industrie, oder bei denen abrasive oder aggressive Substanzen gefördert werden, welche mechanische Lager sehr schnell zerstören würden, beispielsweise Pumpen für Slurry, Schwefel-, Phosphorsäure oder andere Chemikalien in der Halbleiterindustrie.

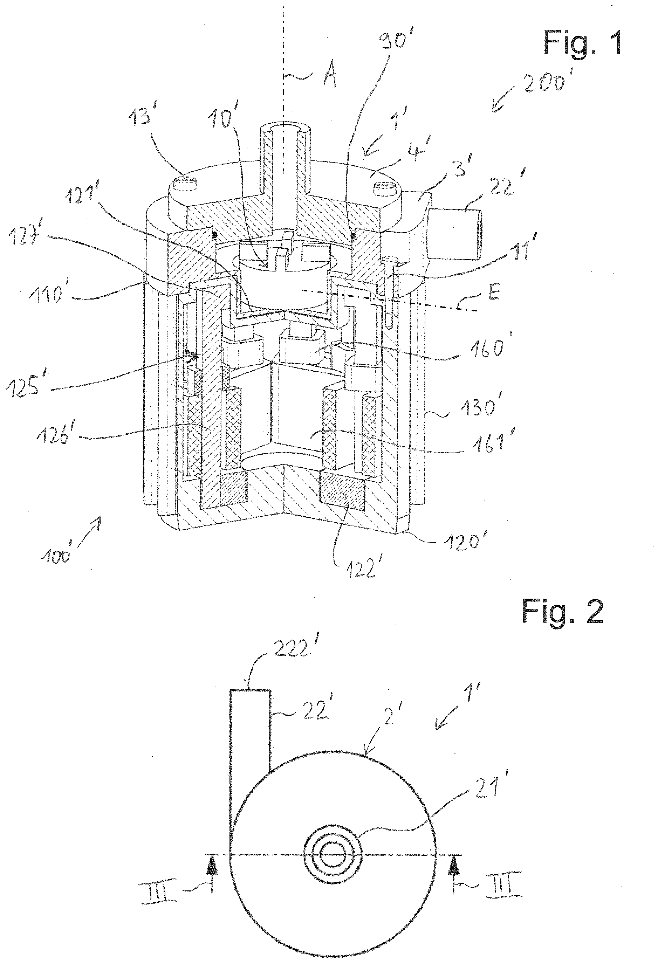

Fig. 1 zeigt eine Darstellung einer aus dem Stand der Technik bekannten Zentrifugalpumpe, die nach dem Prinzip des lagerlosen Motors ausgestaltet ist. Es handelt sich beispielsweise um eine Levitronix^{®} BPS Pumpe. Zum besseren Verständnis ist in Fig. 1 ein Segment herausgeschnitten, damit das Innere der Zentrifugalpumpe sichtbar ist.

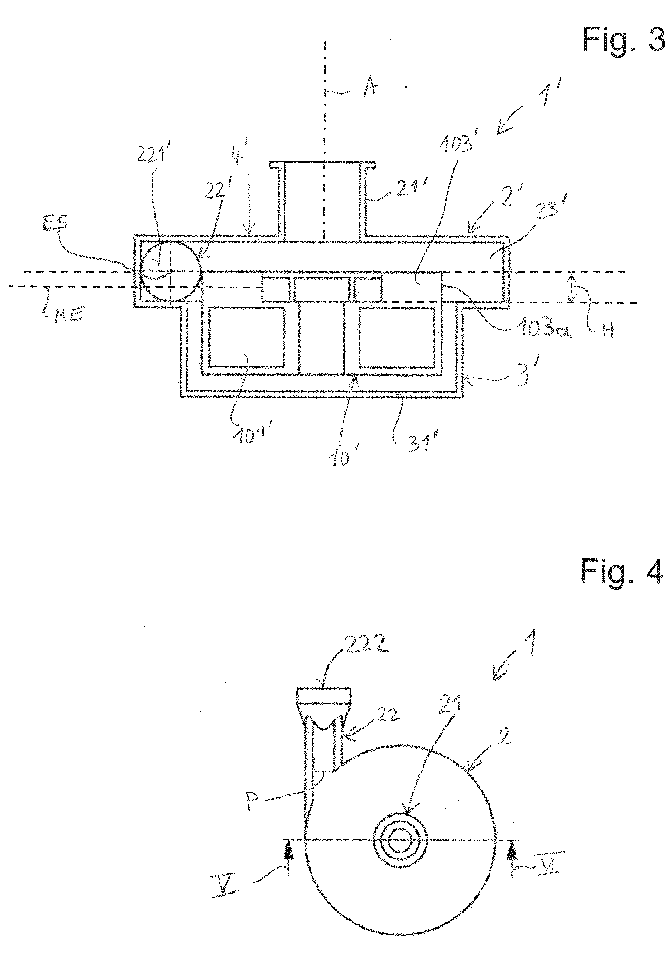

Die Zentrifugalpumpe 200' umfasst einen Stator 100' und eine Pumpeneinheit 1'. Zum besseren Verständnis zeigen Fig. 2 eine Aufsicht auf die Pumpeneinheit 1' aus der axialen Richtung A und Fig. 3 die Pumpeneinheit 1' in einer Schnittdarstellung entlang der Schnittlinie III-III in Fig 2.

Um anzuzeigen, dass es sich bei der Darstellung in Fig. 1, Fig. 2 und Fig. 3 um eine Vorrichtung aus dem Stand der Technik handelt, sind hier die Bezugszeichen jeweils mit einem Hochkomma bzw. mit einem Strich versehen. Die Zentrifugalpumpe ist gesamthaft mit dem Bezugszeichen 200' bezeichnet.

In der Pumpeneinheit 1' ist ein Rotor 10' angeordnet, welcher das Flügelrad bzw. das Laufrad bildet, mit dem das Fluid gefördert wird. Der Stator 100' hat ein Statorgehäuse 130` und erstreckt sich in einer axialen Richtung A von einem ersten axialen Ende 110' bis zu einem zweiten axialen Ende 120`, wobei an dem ersten axialen Ende 110' eine becherförmige Ausnehmung 121' vorgesehen ist, in welche die Pumpeneinheit 1' einsetzbar ist. Der Stator 100' bildet mit dem Rotor 10` einen elektromagnetischen Drehantrieb zum Rotieren des Rotors 10' um die axiale Richtung A. Der Stator 100' ist zur berührungslosen magnetischen Lagerung des Rotors 10` nach dem Prinzip des lagerlosen Motors ausgestaltet. Hierzu ist der Stator 100' als Lager- und Antriebsstator ausgestaltet, mit welchem der Rotor 10' berührungslos magnetisch zur Rotation um die axiale Richtung A antreibbar und berührungslos magnetisch bezüglich des Stators 100' lagerbar ist, wobei der Rotor 10` bezüglich der axialen Richtung A passiv magnetisch stabilisiert ist, und in einer zur axialen Richtung A senkrechten radialen Ebene, die in Fig. 1 durch die Linie E angedeutet ist, aktiv magnetisch gelagert ist.

Der elektromagnetische Drehantrieb mit dem Stator 100' und dem Rotor 10` ist als sogenannter Tempelmotor ausgestaltet. Der Stator 100' umfasst eine Mehrzahl von Spulenkernen 125`, hier acht Spulenkerne 125`, von denen jeder einen Längsschenkel 126` umfasst, welcher sich von einem ersten Ende, in Fig. 1 das darstellungsgemäss untere Ende, in axialer Richtung A bis zu einem zweiten Ende erstreckt, sowie einen Querschenkel 127', welcher an dem zweiten Ende des Längsschenkels 126` und in der radialen Ebene E angeordnet ist. Jeder Querschenkel 127` erstreckt sich von dem zugehörigen Längsschenkel 126` in radialer Richtung auf den Rotor 10' zu und wird durch eine radial innenliegende Stirnfläche begrenzt. Die Spulenkerne 126` sind bezüglich der Umfangsrichtung um die becherförmige Ausnehmung 121' herum angeordnet und damit um den Rotor 10' herum, sodass der Rotor 10` zwischen den radial innenliegenden Stirnflächen der Querschenkel 127` der Spulenkerne 126` angeordnet ist.

Alle ersten Enden der Längsschenkel 126` sind durch einen Rückschluss 122' zur Führung des magnetischen Flusses miteinander verbunden. An jedem Längsschenkel 126` ist mindestens eine konzentrierte Wicklung 160', 161' vorgesehen, welche den jeweiligen Längsschenkel 126` umgibt. Bezüglich Anzahl und Anordnung der konzentrierten Wicklungen 160', 161' sind zahlreiche Varianten bekannt, die hier nicht näher erläutert werden. Es gibt beispielsweise solche Wicklungen 160`, die um genau einen Längsschenkel 126` herum gewickelt sind, und solche Wicklungen 161', die um genau zwei Längsschenkel 126` herum angeordnet sind.

Die Mehrzahl der Längsschenkel 126', die sich in axialer Richtung A erstrecken und an die Säulen eines Tempels erinnern, hat dem Tempelmotor seinen Namen gegeben.

Die Pumpeneinheit 1' (Fig. 2, Fig. 3) umfasst ein Pumpengehäuse 2' mit einem Einlass 21' und mit einem Auslass 22' für das zu fördernde Fluid, sowie den in dem Pumpengehäuse 2' angeordneten Rotor 10' zum Fördern des Fluids, der um die axiale Richtung A rotierbar ist. Das Pumpengehäuse 2` begrenzt einen Pumpenraum 23'. Der Rotor 10` umfasst einen magnetisch wirksamen Kern 101', welcher für die Drehmomentbildung sowie für die Erzeugung der magnetischen Lagerkräfte magnetisch mit dem Stator 100' zusammenwirkt. Der magnetisch wirksame Kern 101' ist beispielsweise ein permanentmagnetischer Ring oder eine permanentmagnetische Scheibe.

Es sind auch solche Ausgestaltungen möglich, bei denen der magnetisch wirksame Kern 101' permanentmagnetfrei, also ohne Permanentmagnete ausgestaltet ist. Der Rotor 10` ist dann z.B. als Reluktanzläufer ausgestaltet. Der magnetisch wirksame Kern 101' des Rotors 10` besteht dann beispielsweise aus einem weichmagnetischen Material. Geeignete weichmagnetische Materialien für den magnetisch wirksamen Kern 101' sind beispielsweise ferromagnetische oder ferrimagnetische Materialien, also insbesondere Eisen, Nickel-Eisen, Kobalt-Eisen, Silizium-Eisen, Mu-Metall.

Ferner sind Ausgestaltungen möglich, bei denen der magnetisch wirksame Kern 101' des Rotors 10' sowohl ferromagnetische Materialien als auch permanentmagnetische Materialien umfasst. Beispielsweise können Permanentmagnete in einen ferromagnetischen Grundkörper eingelegt bzw. eingesetzt werden. Solche Ausgestaltungen sind z.B. vorteilhaft, wenn man bei grossen Rotoren die Kosten durch Einsparen von permanentmagnetischem Material reduzieren will.

Typischerweise ist der magnetisch wirksame Kern 101' mit einem Kunststoff vollständig ummantelt. In anderen Ausführungsformen ist der magnetisch wirksame Kern 101' vollständig in einer Ummantelung eingeschlossen, die aus einem keramischen Material oder aus einem metallischen Material, beispielsweise einem rostfreien Stahl oder Titan oder Tantal, besteht.

Der Rotor 10' umfasst ferner eine Mehrzahl von Flügeln 103' zum Fördern des Fluids vom Einlass 21' zum Auslass 22'.

Das Pumpengehäuse 2` umfasst ein Bodenteil 3' und ein Deckelteil 4' zum Verschliessen des Bodenteils 3`, wobei zwischen dem Bodenteil 3' und dem Deckelteil 4' ein Dichtungselement 90' (Fig. 1) vorgesehen ist, beispielsweise ein O-Ring oder eine Flachdichtung, um eine Leckage des Fluids in die Umgebung zu vermeiden.

Der Einlass 21' des Pumpengehäuses 2` ist in dem Deckelteil 4' angeordnet und so ausgestaltet, dass das zu fördernde Fluid den Rotor 10' in axialer Richtung A anströmt. Der Auslass 22`erstreckt sich parallel zur radialen Ebene E, also im Wesentlichen senkrecht zum Einlass 21'.

Das Bodenteil 3' des Pumpengehäuses 2` weist einen zylindrischen Becher 31' zur Aufnahme des Rotors 10` auf. Der Becher 31' wird in die Ausnehmung 121' im Statorgehäuse 130' eingesetzt, sodass der Rotor 10', genauer gesagt der magnetisch wirksame Kern 101' des Rotors 10` zwischen den Querschenkeln 127` der Spulenkerne 126` angeordnet ist.

Die Pumpeneinheit 1' ist beispielsweise mittels Befestigungselementen 11', z. B. einer Mehrzahl von Schrauben 11', am Statorgehäuse 130` befestigt. Die Schrauben 11' sind an dem Bodenteil 3' angeordnet und fixieren das Bodenteil 3' am ersten axialen Ende 110' des Stators 100'. Das Deckelteil 4' ist üblicherweise über einen Presssitz mit dem Bodenteil 3' verbunden. Zusätzlich wird das Deckelteil 4' mittels mehrerer Befestigungsschrauben 13' am Bodenteil 3' fixiert, welche in axialer Richtung A durch das Deckelteil 4' hindurchgreifen und in das Bodenteil 3' eingreifen.

Für viele Anwendungen, beispielsweise für Anwendungen in der Halbleiterindustrie, wird die Pumpeneinheit 1' - mit Ausnahme des magnetisch wirksamen Kerns 101' - aus einem Kunststoff gefertigt, beispielsweise aus einem Perfluoralkoxy-Polymer (PFA) oder aus Polytetrafluorethylen (PTFE), weil dies Kunststoffe mit einer besonders hohen chemischen Resistenz sind. Diese Kunststoffe sind praktisch inerte Stoffe, die auch von chemisch sehr aggressiven Substanzen, wie sie häufig in der Halbleiterindustrie zum Einsatz kommen, nicht angegriffen werden können. Zudem sind PFA und PTFE sehr reine Kunststoffe, weil sie üblicherweise keine Zusatzstoffe aufweisen und ihre Molekülkomplexe zumindest näherungsweise inert sind. PFA ist häufig bevorzugt, weil es in Spritzgiessverfahren verarbeitet werden kann.

Der Auslass 22' weist eine Eintrittsfläche 221' (Fig. 3) auf, durch welche das Fluid aus dem Pumpenraum 23' in den Auslass 22' strömen kann, sowie eine Austrittsfläche 222' (Fig. 2), durch welche das Fluid aus dem Auslass 22' herausströmen kann. Wie dies am besten in Fig. 3 zu erkennen ist, weist die Eintrittsfläche 221' des Auslasses 22' ein kreisförmiges Profil aus. Die Querschnittsfläche der Eintrittsfläche 221' des Auslasses 22' bestimmt üblicherweise den Druckabfall über den Auslass 22'.

Die Anordnung der Eintrittsfläche 221' des Auslasses 22' bezüglich der axialen Richtung A relativ zu den Flügeln 103' hat einen erheblichen Einfluss auf die berührungslos magnetische Lagerung des Rotors 10', was im Folgenden erläutert wird.

In Fig. 3 ist eine Symmetrieachse des Profils der Eintrittsfläche 221' eingezeichnet, wobei die Symmetrieachse senkrecht auf der axialen Richtung A steht. Auf dieser Symmetrieachse liegt der Schwerpunkt ES des Profils der Eintrittsfläche 221'. Diese Symmetrieachse bildet einen Durchmesser des kreisförmigen Profils der Eintrittsfläche 221', der senkrecht auf der axialen Richtung A steht. Ferner ist eine Mittellinie ME der Flügel 103' eingezeichnet. Die Flügel 103` haben in axialer Richtung A eine Erstreckung, die als ihre Höhe H bezeichnet wird. Die Mittellinie ME der Flügel 103` ist senkrecht zur axialen Richtung A und teilt den jeweiligen Flügel 103' bezüglich der axialen Richtung A in zwei gleich hohe Teile. Die Mittellinien ME aller Flügel 103' liegen in einer Ebene, die senkrecht zur axialen Richtung A ist.

Wenn der in axialer Richtung A gemessene Abstand der Symmetrieachse bzw. des Schwerpunkts ES des Profils der Eintrittsfläche 221' von der Ebene, welche die Mittellinien ME der Flügel 103' enthält grösser wird, so hat dies negative Auswirkungen insbesondere auf die berührungslos magnetische Lagerung des Rotors 10', denn der grössere Abstand führt zu höheren statischen und dynamischen Axial- und Verkippungskräften, die auf den Rotor 10` einwirken. Zudem führt der grössere Abstand zu grösseren radialen Störkräften, die auf den Rotor 10' einwirken und zu einer Reduktion des Wirkungsgrads der Zentrifugalpumpe 200'.

Oft ist es durch die Ausgestaltung des Pumpengehäuses 2` vorgegeben, wie gross der minimale Abstand zwischen der Symmetrieachse bzw. dem Schwerpunkt ES und der Ebene mit den Mittellinien ME ist. Beispielsweise ist es bei solchen Ausgestaltungen des Pumpengehäuses 2', bei denen sowohl der Einlass 21' als auch der Auslass 22' am Deckelteil 4' des Pumpengehäuses 2' angeordnet ist, häufig notwendig, den in axialer Richtung A gemessenen Abstand zwischen der Symmetrieachse bzw. dem Schwerpunkt ES des Profils der Eintrittsfläche 221' und der Ebene mit den Mittellinien ME der Flügel 103` grösser zu machen, weil Platz für die dichtende Verbindung zwischen dem Deckelteil 4' und dem Bodenteil 3' benötigt wird.

Zwar liesse sich der Abstand zwischen der Symmetrieachse bzw. dem Schwerpunkt ES und der Ebene mit den Mittellinien - und damit auch die auf den Rotor 10` wirkenden Störkräfte - dadurch reduzieren, dass man den Durchmesser des Profils der Eintrittsfläche 221' des Auslasses 22' verkleinert, aber damit würde man den Druckabfall über den Auslass 22' vergrössern, was unerwünscht ist.

Ausgehend von diesem Stand der Technik ist es daher eine Aufgabe der Erfindung, eine Pumpeneinheit mit einem berührungslos magnetisch lagerbaren Rotor für eine Zentrifugalpumpe vorzuschlagen, bei welcher die auf den Rotor wirkenden Störkräfte reduziert werden. Zudem ist es eine Aufgabe der Erfindung, eine Zentrifugalpumpe mit einer solchen Pumpeneinheit vorzuschlagen.

Der diese Aufgabe lösende Gegenstand der Erfindung ist durch die Merkmale des unabhängigen Patentanspruchs gekennzeichnet.

Erfindungsgemäss wird also eine Pumpeneinheit für eine Zentrifugalpumpe vorgeschlagen, welche die Pumpeneinheit und einen Stator umfasst, der sich in einer axialen Richtung von einem ersten axialen Ende bis zu einem zweiten axialen Ende erstreckt, wobei an dem ersten axialen Ende eine becherförmige Ausnehmung vorgesehen ist, in welche die Pumpeneinheit einsetzbar ist, wobei die Pumpeneinheit ein Pumpengehäuse mit einem Einlass und mit einem Auslass für ein zu förderndes Fluid aufweist, sowie einen in dem Pumpengehäuse angeordneten Rotor mit einer Mehrzahl von Flügeln zum Fördern des Fluids, wobei das Pumpengehäuse einen Pumpenraum begrenzt, wobei der Rotor um die axiale Richtung rotierbar ist, wobei die Pumpeneinheit für eine berührungslos magnetische Lagerung des Rotors und für einen berührungslos magnetischen Antrieb des Rotors durch den Stator ausgestaltet ist, wobei das Pumpengehäuse ein Deckelteil und ein aufweist, wobei das Bodenteil einen zylindrischen Becher zur Aufnahme des Rotors aufweist, welcher Becher in die becherförmige Ausnehmung des Stators einsetzbar ist, und wobei der Auslass eine Eintrittsfläche aufweist, durch welche das Fluid aus dem Pumpenraum in den Auslass strömen kann. Die Eintrittsfläche des Auslasses weist ein Profil auf, welches verschieden von einer Kreisfläche ist.

Durch diese Ausgestaltung der Eintrittsfläche des Auslasses kann der in axialer Richtung gemessene Abstand zwischen der Eintrittsfläche und den Flügeln deutlich reduziert werden, ohne dass die Querschnittsfläche der Eintrittsfläche reduziert werden muss. Da das Profil der Eintrittsfläche des Auslasses ein Profil ist, welches von einer Kreisfläche verschieden ist, kann die Eintrittsfläche bezüglich der axialen Richtung näher an den Flügeln angeordnet werden als bei einem kreisförmigen Profil der Eintrittsfläche, ohne die Querschnittsfläche der Eintrittsfläche zu reduzieren.

Vorzugsweise weist das Profil der Eintrittsfläche eine Profilhöhe in axialer Richtung auf, wobei es mindestens eine zweite Richtung gibt, die mit der axialen Richtung einen von null verschiedenen Winkel einschliesst, und wobei die maximale Erstreckung des Profils in der zweiten Richtung grösser ist als die Profilhöhe. Das bedeutet, dass das Profil in irgendeiner zweiten Richtung, die verschieden ist von der axialen Richtung, eine maximale Erstreckung hat, welche grösser ist als die maximale Erstreckung des Profils in der axialen Richtung, also die Profilhöhe.

Falls die Profilhöhe über das Profil gesehen nicht konstant ist, so bezeichnet die Profilhöhe die maximale Erstreckung des Profils in der axialen Richtung

Bevorzugt sind solche Ausgestaltungen, bei denen die Eintrittsfläche des Auslasses ein Profil mit mehreren geradlinigen Kanten aufweist.

Besonders bevorzugt beträgt der Winkel zwischen der zweiten Richtung und der axialen Richtung 90°. Speziell bevorzugt ist die zweite Richtung dann eine radiale Richtung, die senkrecht auf der axialen Richtung steht. Die Erstreckung in der zweiten Richtung ist dann die Profilbreite, welche die maximale Erstreckung des Profils in radialer Richtung ist.

Vergleicht man beispielsweise eine Eintrittsfläche mit einem kreisförmigen Profil, das einen festgelegten Durchmesser aufweist, mit einer erfindungsgemäss ausgestalteten Eintrittsfläche, so kann die erfindungsgemäss ausgestaltete Eintrittsfläche mit einer grösseren Querschnittsfläche ausgestaltet werden als die Eintrittsfläche mit einem kreisförmigen Profil, ohne dass die Profilhöhe, also die maximale Erstreckung der erfindungsgemäss ausgestalteten Eintrittsfläche in axialer Richtung grösser ist als der festgelegte Durchmesser der Eintrittsfläche mit dem kreisförmigen Profil.

Umgekehrt bedeutet dies, dass die erfindungsgemäss ausgestaltete Eintrittsfläche im Vergleich zu einer Eintrittsfläche mit kreisförmigem Profil mit der gleichen Querschnittsfläche aber mit einer kleineren Erstreckung insbesondere in axialer Richtung ausgestaltet werden kann. Somit kann die erfindungsgemäss ausgestaltete Eintrittsfläche bezüglich der axialen Richtung näher an bzw. mit grösserem Überlapp mit den Flügeln angeordnet werden. Dies wirkt sich besonders vorteilhaft auf die berührungslos magnetische Lagerung des Rotors aus, weil die statischen und dynamischen Axial- und Verkippungskräfte auf den Rotor sowie die in radialer Richtung auf den Rotor wirkenden Störkräfte deutlich reduziert werden.

Gemäss einer vorteilhaften Ausführungsform weist das Profil der Eintrittsfläche des Auslasses mindestens eine und vorzugsweise mehrere geradlinige Kanten auf. weist.

In einer besonders bevorzugten Ausgestaltung weist das Profil der Eintrittsfläche genau vier geradlinige Kanten auf.

Dabei ist das Profil der Eintrittsfläche beispielsweise im Wesentlichen rechteckförmig oder quadratisch ausgestaltet.

Ferner ist es eine insbesondere aus fertigungstechnischen Gründen bevorzugte Ausführungsform, dass zwei benachbarte geradlinige Kanten des Profils durch eine Abrundung verbunden sind.

Vorzugsweise weist jeder Flügel eine Austrittskante auf, sowie eine zur axialen Richtung senkrechte Mittellinie, welche den jeweiligen Flügel an der Austrittskante bezüglich der axialen Richtung in zwei gleich hohe Abschnitte teilt, und wobei die Mittellinien aller Flügel in einer Ebene liegen. Mit der Austrittskante des Flügels ist dabei das Ende des jeweiligen Flügels gemeint, an welchem das Fluid das Flügelrad verlässt. Üblicherweise ist die Austrittskante des Flügels seine bezüglich der radialen Richtung aussenliegende Kante.

Gemäss einer bevorzugten Ausgestaltung weist das Profil der Eintrittsfläche einen Schwerpunkt auf, wobei jeder Flügel an der Austrittskante eine Höhe in axialer Richtung hat, und wobei der Schwerpunkt des Profils der Eintrittsfläche einen Abstand von der Ebene aufweist, in welcher die Mittellinien der Flügel liegen, der höchsten das 1.5-fache der Höhe (H) der Flügel (103) an der Austrittskante (103a) beträgt. Falls das Profil der Eintrittsfläche symmetrisch ist, beispielsweise rechteckig, so liegt der Schwerpunkt des Profils der Eintrittsfläche auf dem Schnittpunkt der Symmetrieachsen. Es ist jedoch keinesfalls notwendig, dass das Profil der Eintrittsfläche symmetrisch ist. Deshalb wird hier auf den Schwerpunkt des Profils der Eintrittsfläche Bezug genommen..

Es sind Ausgestaltungen möglich, bei welchen die Höhe jedes Flügels über den gesamten Flügel konstant ist. Es sind aber auch Ausgestaltungen möglich, bei welchen sich die Höhe des Flügels ändert, beispielsweise kann der Flügel an seiner Eintrittskante eine grössere Höhe aufweisen als an seiner Austrittskante.

Speziell bevorzugt ist es, dass der Schwerpunkt des Profils der Eintrittsfläche einen Abstand von der Ebene aufweist, in welcher die Mittellinien der Flügel liegen, der höchstens das 1.1-fache der Höhe (H) der Flügel (103) an der Austrittskante (103a) beträgt.

Gemäss einer bevorzugten Ausführungsform sind sowohl der Einlass als auch der Auslass des Pumpengehäuses am Deckelteil angeordnet.

In einer vorteilhaften Ausführungsform weist der Auslass eine Austrittsfläche auf, durch welche das Fluid aus dem Auslass herausströmen kann, wobei die Austrittsfläche als Kreisfläche ausgestaltet ist.

Dabei ist es bevorzugt, dass die Austrittsfläche eine grössere Querschnittsfläche aufweist als die Eintrittsfläche.

Vorzugsweise ist der Auslass in Strömungsrichtung gesehen sich konisch erweiternd ausgestaltet. Dadurch kann der Auslass als Diffusor ausgestaltet werden, mit welchem kinetische Energie des Fluids in potentielle Energie umgewandelt werden kann.

Eine weitere bevorzugte Massnahme besteht darin, dass der Auslass einen flachen Boden aufweist, der zum Anliegen an dem ersten axialen Ende des Stators ausgestaltet ist.

Durch die Erfindung wird ferner eine Zentrifugalpumpe zum Fördern eines Fluids vorgeschlagen mit einer erfindungsgemäss ausgestalteten Pumpeneinheit, und mit einem Stator, der sich in einer axialen Richtung von einem ersten axialen Ende bis zu einem zweiten axialen Ende erstreckt, wobei an dem ersten axialen Ende eine becherförmige Ausnehmung vorgesehen ist, in welche der zylindrischen Becher der Pumpeneinheit einsetzbar ist, wobei der Stator mit dem Rotor einen elektromagnetischen Drehantrieb zum Rotieren des Rotors um die axiale Richtung bildet, wobei der Stator als Lager- und Antriebsstator ausgestaltet ist, mit welchem der Rotor berührungslos magnetisch antreibbar und berührungslos magnetisch bezüglich des Stators lagerbar ist, wobei der Rotor bezüglich der axialen Richtung passiv magnetisch stabilisiert ist, und in einer zur axialen Richtung senkrechten radialen Ebene aktiv magnetisch gelagert ist.

Besonders bevorzugt ist der elektromagnetische Drehantrieb als Tempelmotor ausgestaltet, wobei der Stator eine Mehrzahl von Spulenkernen aufweist, von denen jeder einen Längsschenkel umfasst, welcher sich von einem ersten Ende in axialer Richtung bis zu einem zweiten Ende erstreckt, sowie einen Querschenkel, welcher an dem zweiten Ende des Längsschenkels und in der radialen Ebene angeordnet ist, und welcher sich von dem Längsschenkel in radialer Richtung erstreckt, wobei die Spulenkerne bezüglich der Umfangsrichtung um den Rotor herum angeordnet sind, sodass der Rotor zwischen den Querschenkeln der Spulenkerne angeordnet ist, und wobei an jedem Längsschenkel mindestens eine konzentrierte Wicklung vorgesehen ist, welche den jeweiligen Längsschenkel umgibt.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der schematischen Zeichnung zeigen (teilweise im Schnitt):
- Fig. 1:: eine perspektivische Darstellung einer Zentrifugalpumpe gemäss Stand der Technik, teilweise im Schnitt,
- Fig. 2:: eine Aufsicht auf die Pumpeneinheit aus der axialen Richtung A,
- Fig. 3: die Pumpeneinheit aus Fig. 2 in einer Schnittdarstellung entlang der Schnittlinie III-III in Fig 2,
- Fig. 4:: eine Aufsicht auf ein erstes Ausführungsbeispiel einer erfindungsgemässen Pumpeneinheit,
- Fig. 5:: das erste Ausführungsbeispiel in einer Schnittdarstellung entlang der Schnittlinie V-V in Fig. 4,
- Fig. 6 - 9:: verschiedene Varianten für das erste Ausführungsbeispiel, jeweils in einer Darstellung, die derjenigen aus Fig. 5 entspricht,
- Fig. 10:: ein zweites Ausführungsbeispiel einer erfindungsgemässen Pumpeneinheit in einer Schnittdarstellung entlang der axialen Richtung,
- Fig. 11:: ein drittes Ausführungsbeispiel einer erfindungsgemässen Pumpeneinheit in einer Schnittdarstellung entlang der axialen Richtung,
- Fig. 12:: ein viertes Ausführungsbeispiel einer erfindungsgemässen Pumpeneinheit in einer Schnittdarstellung entlang der axialen Richtung,
- Fig. 13:: eine perspektivische Darstellung eines fünften Ausführungsbeispiels einer erfindungsgemässen Pumpeneinheit,
- Fig. 14:: das fünfte Ausführungsbeispiel in einer Schnittdarstellung,
- Fig. 15 - 16:: weitere Variante für das Profil der Eintrittsfläche des Auslasses, und
- Fig. 17:: eine schematische Schnittdarstellung eines Ausführungsbeispiels einer erfindungsgemässen Zentrifugalpumpe.

Wie bereits vorangehend erläutert, zeigt Fig. 1 eine Zentrifugalpumpe 200' mit einem berührungslos magnetisch gelagerten und berührungslos magnetisch angetriebenen Rotor 10', die aus dem Stand der Technik bekannt ist. Fig. 2 und Fig. 3 zeigen in einer Aufsicht bzw. in einer Schnittdarstellung die Pumpeneinheit 1' dieser Zentrifugalpumpe 200'.

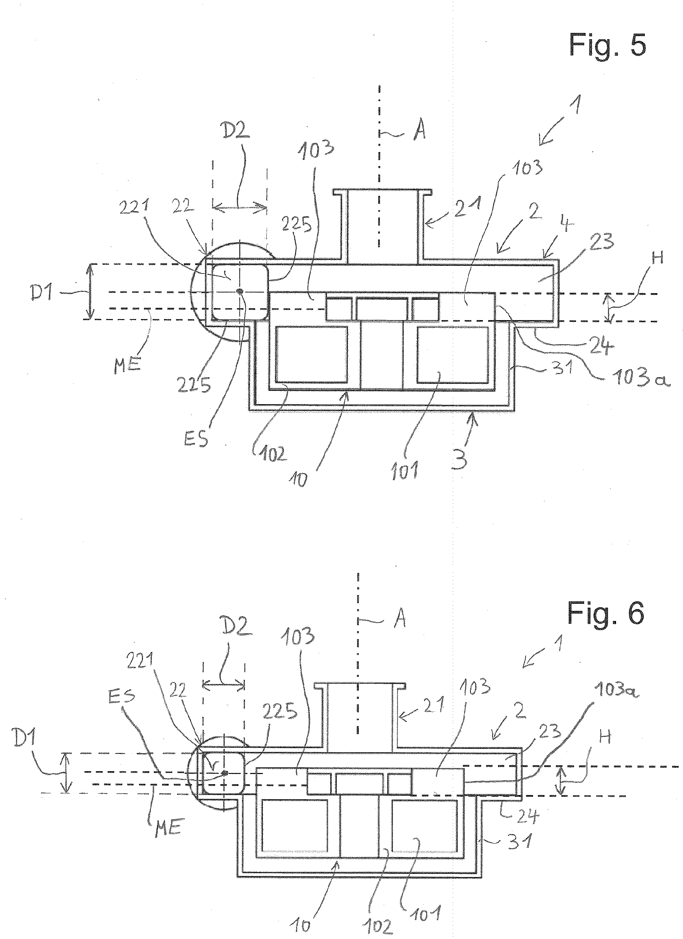

Fig. 4 zeigt in einer der Fig. 2 entsprechenden Darstellung eine Aufsicht auf ein erstes Ausführungsbeispiel einer erfindungsgemässen Pumpeneinheit, die gesamthaft mit dem Bezugszeichen 1 bezeichnet ist. Zum besseren Verständnis zeigt Fig. 5 das erste Ausführungsbeispiel der Pumpeneinheit 1 noch in einer der Fig. 3 entsprechenden Schnittdarstellung. Der Schnitt erfolgt entlang der Schnittlinie V-V in Fig. 4.

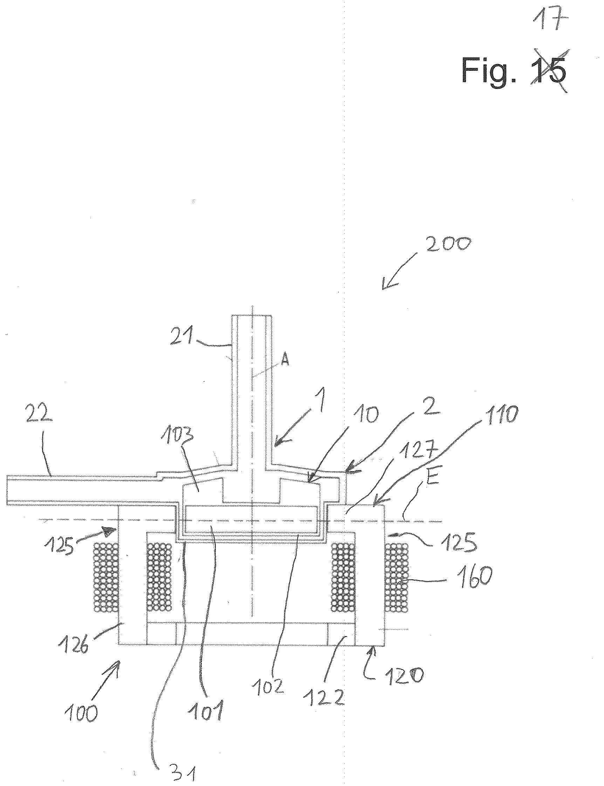

Die Pumpeneinheit 1 ist für eine Zentrifugalpumpe 200 (siehe Fig. 17) zum Fördern eines Fluids ausgestaltet und umfasst ein Pumpengehäuse 2 mit einem Einlass 21 und mit einem Auslass 22 für das Fluid. In dem Pumpengehäuse 2 ist ein Rotor 10 zum Fördern des Fluids angeordnet, welcher das Flügelrad bzw. das Laufrad der Pumpeneinheit 1 und damit der Zentrifugalpumpe 200 bildet. Der Rotor 10 ist um eine Solldrehachse rotierbar, welche eine axiale Richtung A definiert.

Eine zur axialen Richtung A senkrechte Richtung wird als radiale Richtung bezeichnet. Im Folgenden wird der Ausdruck "axial" mit der allgemein üblichen Bedeutung "in axialer Richtung" oder "bezüglich der axialen Richtung" verwendet. Der Begriff "radial" wird mit der allgemein üblichen Bedeutung "in radialer Richtung" oder "bezüglich der axialen Richtung" verwendet

Die Pumpeneinheit 1 ist für eine berührungslos magnetische Lagerung des Rotors 10 und für einen berührungslos magnetischen Antrieb des Rotors 10 ausgestaltet. Dies kann insbesondere in sinngemäss gleicher Weise realisiert sein, wie dies anhand der Fig. 1 bis Fig. 3 erläutert ist. So kann die erfindungsgemässe Pumpeneinheit 1 bezüglich der magnetischen Lagerung und des magnetischen Antriebs in sinngemäss gleicher Weise ausgestaltet sein wie die Pumpeneinheit 1' in Fig. 3. Dazu umfasst der Rotor 10 der Pumpeneinheit 1 einen magnetisch wirksamen Kern 101, der beispielsweise als permanentmagnetische Ring oder permanentmagnetische Scheibe ausgestaltet ist, und von einer Kunststoffummantelung 102 umschlossen wird. Die Kunststoffummantelung 102 besteht beispielsweise aus PTFE oder PFA.

Der Rotor 10 umfasst ferner eine Mehrzahl von Flügeln 103, um das Fluid vom Einlass 21 zum Auslass 22 zu fördern. Jeder Flügel 103 erstreckt sich von einer radial innenliegenden Eintrittskante bis zu einer radial aussenliegenden Austrittskante 103a, an welcher das Fluid aus dem Rotor 10 austritt. Die Flügel 103 sind auf der Kunststoffummantelung 102 des magnetisch wirksamen Kerns 101 angeordnet. Die Flügel 103 bestehen vorzugsweise aus Kunststoff und können beispielsweise einstückig mit der Kunststoffummantelung 102 ausgestaltet sein. Natürlich ist es auch möglich, die individuellen Flügel 103 oder die Gesamtheit der Flügel 103 in einem separaten Fertigungsprozess herzustellen und sie dann mit der Kunststoffummantelung 102 des magnetisch wirksamen Kerns 101 zu verbinden, beispielsweise mittels eines Schweissprozesses.

Das von dem Rotor 10 gebildete Laufrad mit den Flügeln 103 ist vorzugsweise als radiales Laufrad ausgestaltet, welches von dem Fluid vom Einlass 21 in axialer Richtung A angeströmt wird, und das Fluid dann in eine radiale Richtung umlenkt.

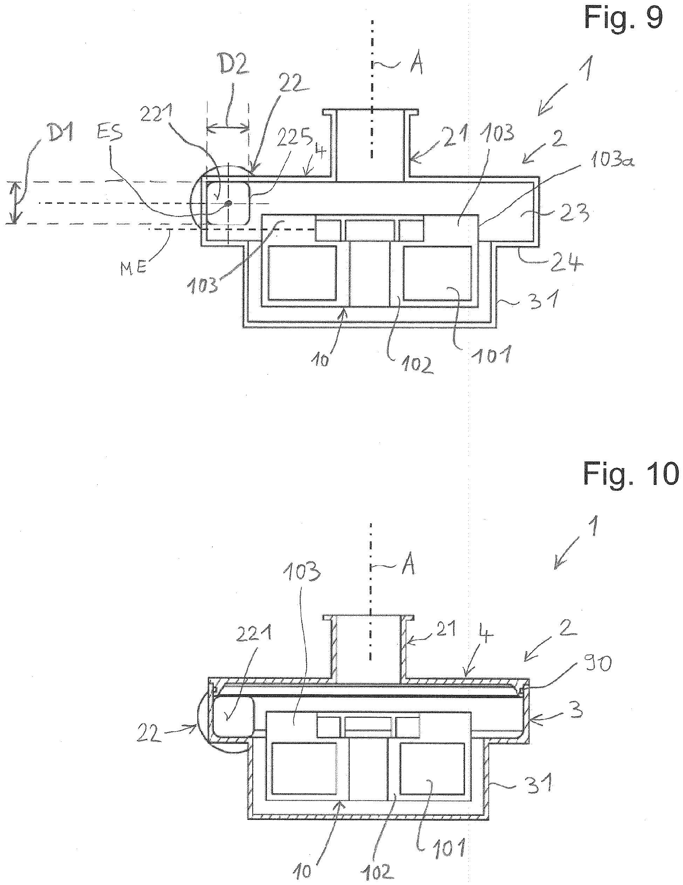

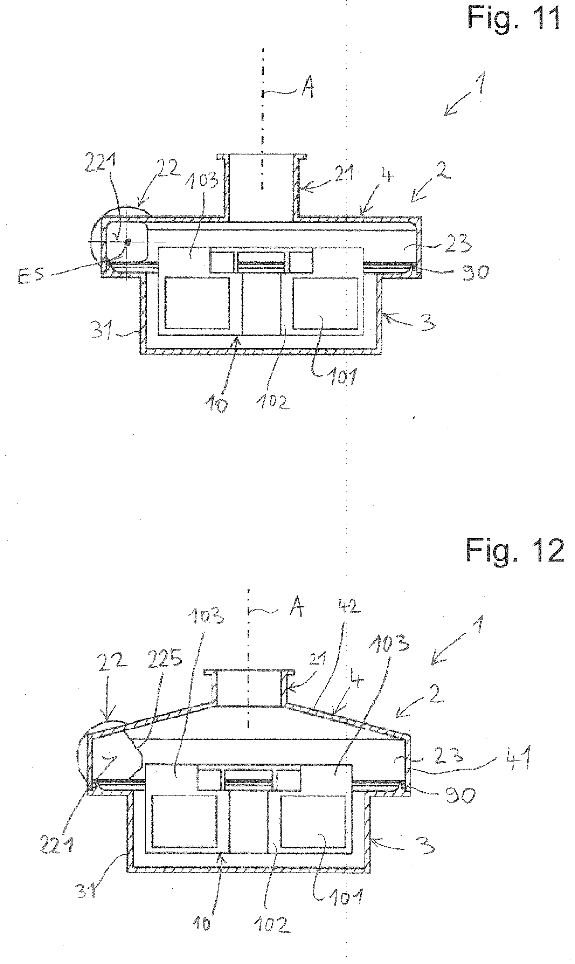

Das Pumpengehäuse 2 umfasst ein Deckelteil 4 und ein Bodenteil 3, die dichtend miteinander verbunden werden, was weiter hinten anhand der Fig. 10 und Fig. 11 noch näher erläutert wird. Das Bodenteil 3 umfasst einen zylindrischen Becher 31 zur Aufnahme des Rotors 10. Der Becher 31 ist vorzugsweise so ausgestaltet und angeordnet, dass er in eine becherförmige Ausnehmung eines Stators 100 einsetzbar ist wie dies in Fig. 17 schematisch dargestellt ist.

Der Stator 100 (Fig. 17) erstreckt sich in der axialen Richtung A von einem ersten axialen Ende 110 bis zu einem zweiten axialen Ende 120 und weist ein Statorgehäuse (in Fig. 17 nicht dargestellt) auf, das im Wesentlichen zylindrisch ausgestaltet ist. Die becherförmige Ausnehmung ist an dem ersten axialen Ende 110 des Stators 100 angeordnet, vorzugsweise zentral in der Stirnfläche, welche das erste axiale Ende 110 des Stators 100 bildet. Die Ausgestaltung des Statorgehäuses, mit der becherförmigen Ausnehmung kann insbesondere in analoger Weise realisiert sein, wie dies anhand der Fig. 1 für das Statorgehäuse 130' und die becherförmige Ausnehmung 121' erläutert wurde. Der Becher 31 ist dann also derart angeordnet und ausgestaltet, dass er in die Ausnehmung 121` (Fig. 1) im ersten axialen Ende 110' des Stators 100' einsetzbar ist, und der magnetisch wirksame Kern 101 zwischen den Querschenkeln 127` der Spulenkerne 125' angeordnet ist.

Wie dies in Fig. 5 zu erkennen ist, befindet sich darstellungsgemäss oberhalb des Bechers 31 ein Pumpenraum 23, welcher durch das Pumpengehäuse 2 begrenzt wird, und in welchem die Flügel 103 des Rotors 10 angeordnet sind. Der Pumpenraum 23 ist zumindest im Wesentlichen zylindrisch ausgestaltet, wobei der Durchmesser des Pumpenraums 23 grösser ist als der Innendurchmesser des Bechers 31. Hierdurch weist das Pumpengehäuse 2 einen flanschartigen Vorsprung 24 auf, welcher den Pumpenraum 23 bezüglich der axialen Richtung A darstellungsgemäss nach unten begrenzt.

Der Einlass 21 ist zentral im Deckelteil 3 des Pumpengehäuses 2 angeordnet, sodass das Fluid den Rotor 10 in axialer Richtung A anströmen kann.

Der Auslass 22 weist eine Eintrittsfläche 221 auf, durch welche das Fluid aus dem Pumpenraum 23 in den Auslass 22 einströmen kann, sowie eine Austrittsfläche 222 (Fig. 4), durch welche das Fluid den Auslass 22 verlässt. Mit dem Profil der Eintrittsfläche 221 bzw. der Austrittsfläche 222 wird wie allgemein üblich, die Querschnittsfläche senkrecht zur Hauptströmungsrichtung des Fluids im Auslass 22 bezeichnet. Das Profil der Eintrittsfläche 221 ist in Fig. 4 durch die Linie mit dem Bezugszeichen P angedeutet. Das Profil der Eintrittsfläche 221 ist die senkrechte Projektion der Eintrittsfläche 221 auf eine Ebene, die senkrecht auf der Strömungsrichtung des Fluids im Auslass 22 steht.

Erfindungsgemäss ist die Eintrittsfläche 221 des Auslasses 22 so ausgestaltet, dass ihr Profil von einer Kreisfläche verschieden ist. Gemäss einer bevorzugten Ausführungsform weist das Profil der Eintrittsfläche 221 mindestens eine geradlinige Kante 225 auf. Vorzugsweise weist das Profil eine Mehrzahl von geradlinigen Kanten 225 auf. Im Unterschied zu bekannten Ausgestaltungen (siehe z. B. Fig. 3) ist also die Eintrittsfläche 221 nicht mit einem kreisförmigen Profil ausgestaltet, sondern mit einem Profil, das beispielsweise mindestens eine und vorzugsweise mehrere geradlinige Kante(n) 225 aufweist. Dies hat den Vorteil, dass die Querschnittsfläche der Eintrittsfläche 221 grösser ausgestaltet werden kann als bei einem kreisförmigen Profil, ohne dass dafür die Erstreckung in axialer Richtung A vergrössert werden muss, oder dass die Eintrittsfläche 221 des Auslasses 22 bezüglich der axialen Richtung A näher an die Flügel 103 bzw. mit einem grösseren Überlapp mit den Flügeln 103 angeordnet werden kann. Dies wird weiter hinten noch erläutert.

Die Austrittsfläche 222 des Auslasses 22 (Fig. 4), durch welche das Fluid aus dem Auslass 22 herausströmen kann, ist vorzugsweise als Kreisfläche ausgestaltet. Der Auslass 22 geht also über von der Eintrittsfläche 221, deren Profil mit mehreren geradlinigen Kanten 225 ausgestaltet ist, in die Austrittsfläche 222, die ein kreisförmiges Profil aufweist. Vorzugsweise ist die Querschnittsfläche der Austrittsfläche grösser ausgestaltet als die Querschnittsfläche der Eintrittsfläche 221, sodass der Auslass 22 als Diffusor ausgestaltet werden kann.

Der Auslass 22 ist an seinem Ende, an welchem die Austrittsfläche 222 angeordnet ist, vorzugsweise derart ausgestaltet, dass er einen runden Auslassanschluss bildet, der mit einer Leitung oder mit einem Schlauch verbunden werden kann.

Bei dem in Fig. 5 gezeigten ersten Ausführungsbeispiel der Pumpeneinheit 1 ist die Eintrittsfläche 221 mit einem im Wesentlichen rechteckförmigen, insbesondere mit einem im Wesentlichen quadratischen Profil ausgestaltet, das heisst das Profil der Eintrittsfläche 221 weist genau vier geradlinige Kanten 225 auf, die paarweise parallel zueinander angeordnet sind.

Aus fertigungstechnischen Gründen ist es dabei bevorzugt, dass zwei benachbarte geradlinige Kanten 225 jeweils durch eine Abrundung verbunden sind. Somit hat das Profil der Eintrittsfläche 221 hier die Form eines Rechtecks, bzw. eines Quadrats mit abgerundeten Ecken.

Die maximale Erstreckung des Profils der Eintrittsfläche 221 in axialer Richtung A wird im Folgenden als Profilhöhe D1 bezeichnet, und die maximale Erstreckung in der dazu senkrechten radialen Richtung als Profilbreite D2.

Bei dem hier beschriebenen ersten Ausführungsbeispiel der Pumpeneinheit 1 hat das Profil der Eintrittsfläche 221 eine Symmetrieachse, welche senkrecht auf der axialen Richtung A steht. Da das Profil der Eintrittsfläche 221 aber auch so ausgestaltet sein kann, dass es keine Symmetrieachse aufweist, wird im Folgenden auf den Schwerpunkt ES des Profils der Eintrittsfläche 221 Bezug genommen. Im Falle einer symmetrischen Ausgestaltung des Profils der Eintrittsfläche, beispielsweise bei einem rechteckförmigen Profil, ist der Schwerpunkt ES der Schnittpunkt der Symmetrieachsen des Rechtecks.

Die Flügel 103, mit denen das Fluid in den Auslass 22 gefördert wird, haben eine Höhe H, womit die Erstreckung der Flügel 103 in axialer Richtung A bezeichnet wird. Da die Flügel in radialer Richtung gesehen keine konstante Höhe H aufweisen müssen (siehe z.B. Fig. 17), wird im Folgenden mit der Höhe H der Flügel 103 die Höhe H des Flügels 103 an der Austrittskante 103a des Flügels 103 bezeichnet. Ferner hat jeder Flügel 103 eine zur axialen Richtung A senkrechte Mittellinie ME, welche den jeweiligen Flügel 103 an der Austrittskante 103a bezüglich der axialen Richtung A in zwei gleich hohe Abschnitte teilt. Die Mittellinien ME aller Flügel 103 liegen alle in der gleichen Ebene, die senkrecht zur axialen Richtung A ist.

Wenn man die aus dem Stand der Technik bekannte Ausgestaltung der Eintrittsfläche 221', die in Fig. 3 dargestellt ist, mit der Eintrittsfläche 221 des ersten Ausführungsbeispiels, das in Fig. 5 dargestellt ist, vergleicht, so ist zu erkennen, dass die Querschnittsfläche des kreisförmigen Profils der Eintrittsfläche 221' in Fig. 3 kleiner ist als die Querschnittsfläche des rechteckigen Profils der Eintrittsfläche 221 in Fig. 5, obwohl beide Profile die gleiche maximale Erstreckung haben. Das kreisförmige Profil (Fig. 3) hat beispielsweise einen Durchmesser, der gleich gross ist wie die Profilhöhe D1 des Profils der Eintrittsfläche 221 in Fig. 5. Daraus ergibt sich, dass die Querschnittsfläche des Profils in Fig. 5 grösser ist als die Querschnittsfläche des Profils in Fig. 3, wobei beide Profile die gleiche maximale Erstreckung in der axialen Richtung A haben.

Da die Pumpengehäuse 2' bzw. 2 in Fig. 3 bzw. Fig. 5 ansonsten identisch ausgestaltet sein können, ist es mit der erfindungsgemässen Ausgestaltung möglich, die Eintrittsfläche 221 des Auslasses 22 mit einer grösseren Querschnittsfläche auszugestalten als bei der aus dem Stand der Technik bekannten Lösung (Fig. 3), ohne dass dafür die geometrischen Abmessungen des Pumpengehäuses 2 geändert werden müssen. Eine grössere Querschnittsfläche der Eintrittsfläche 221 des Auslasses 22 hat den Vorteil, dass der Druckabfall über den Auslass 22, der im Wesentlichen durch diese Querschnittsfläche bestimmt wird, reduziert werden kann, was im Hinblick auf die Effizienz der Pumpeneinheit 1 vorteilhaft ist.

Will man aber andererseits die Querschnittsfläche der Eintrittsfläche 221 des Auslasses 22 im Vergleich zu der bekannten und in Fig. 3 dargestellten kreisförmigen Ausgestaltung nicht vergrössern, so kann die Eintrittsfläche 221 mit einer reduzierten Profilhöhe D1 und/oder mit einer reduzierten Profilbreite D2 ausgestaltet werden.

Eine solche Variante des ersten Ausführungsbeispiels der erfindungsgemässen Pumpeneinheit 1 ist in Fig. 6 dargestellt. Bei der in Fig. 6 dargestellten Variante ist das Profil der Eintrittsfläche 221 so ausgestaltet, dass die Querschnittsfläche des Profils etwa gleich gross ist wie die kreisförmige Querschnittsfläche der Eintrittsfläche 221' in Fig. 3. Wenn diese beiden Querschnittsflächen gleich gross sein sollen, so bedeutet dies, dass die Profilhöhe D1 kleiner ist als der Durchmesser der kreisförmigen Querschnittsfläche in Fig. 3. Dies hat einerseits zur Folge, dass der Pumpenraum 23 mit einer geringeren Höhe in axialer Richtung A ausgestaltet werden kann.

Andererseits kann die Eintrittsfläche 221 bezüglich der axialen Richtung A näher an den Flügeln 103 bzw. mit einem grösseren Überlapp mit den Flügeln 103 angeordnet werden. Dies erkennt man beispielsweise daran, dass der Schwerpunkt ES des Profils der Eintrittsfläche 221 bei der Variante gemäss Fig. 6 einen geringeren Abstand von der Ebene hat, in welcher die Mittellinien ME der Flügel 103 liegen, als bei der in Fig. 5 dargestellten Ausführungsform. Die Mitte des Auslasses 22 (bezüglich der axialen Richtung A) liegt bei der Variante gemäss Fig. 6 darstellungsgemäss tiefer und somit näher an den Flügeln 103 des Rotors 10 als bei der in Fig. 5 dargestellten Ausführungsform.

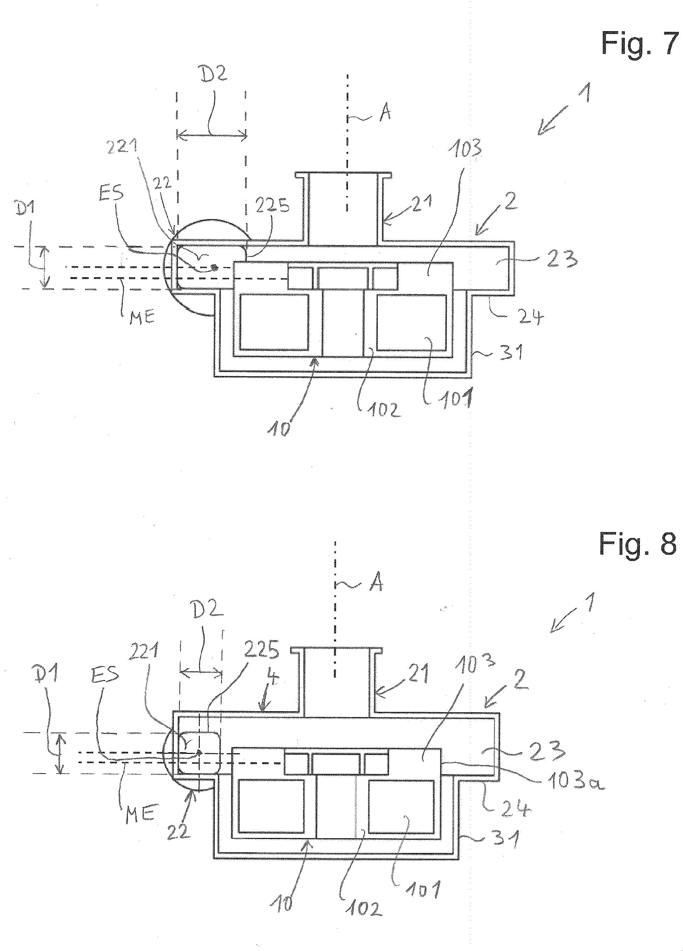

Bei der in Fig. 7 dargestellten Variante ist das Profil der Eintrittsfläche 221 im Wesentlichen rechteckförmig ausgestaltet, wobei die Profilbreite D2 grösser ist als die Profilhöhe D1. Bei der in Fig. 7 dargestellten Variante überlappen sich die Eintrittsfläche 221 und die Flügel 103 sowohl in axialer Richtung A als auch in radialer Richtung. Mit der in Fig. 7 dargestellten Variante lässt sich die Querschnittsfläche der Eintrittsfläche 221 vergrössern, ohne dass dafür die in axialer Richtung A gemessene Höhe der Pumpenkammer 23 vergrössert werden muss.

Durch die rechteckige Ausgestaltung, bei welcher die Profilbreite D2 grösser ist als die Profilhöhe D1, kann die Eintrittsfläche 221 bezüglich der axialen Richtung A darstellungsgemäss weiter unten angeordnet werden. Folglich reduziert sich der Abstand des Schwerpunkts ES von der Ebene, in welcher die Mittellinien ME der Flügel 103 liegen, was vorteilhaft für die magnetische Lagerung des Rotors 10 ist.

In den Fig. 8 und Fig. 9 sind zwei Varianten gezeigt, bei welchen die Eintrittsflächen 221 mit einer Profilhöhe D1 ausgestaltet, die kleiner ist als die Höhe der Pumpenkammer 23, womit die Erstreckung der Pumpenkammer 23 in axialer Richtung A gemeint ist. Dabei sind Ausgestaltungen möglich (siehe Fig. 8), bei welchen die Eintrittsfläche 221 bezüglich der axialen Richtung A unmittelbar benachbart bzw. angrenzend an den flanschartigen Vorsprung 24 und beabstandet vom Deckelteil 4 des Pumpengehäuses 2 angeordnet sind. Es sind aber auch Ausgestaltungen möglich (siehe Fig. 9), bei welchen die Eintrittsfläche 221 bezüglich der axialen Richtung A unmittelbar benachbart bzw. angrenzend an das Deckelteil 4 des Pumpengehäuses 2 und beabstandet vom flanschartigen Vorsprung 24 angeordnet sind.

Es versteht sich, dass die in den Fig. 5-9 gezeigten Ausgestaltungen beispielhaften Charakter haben. Die Profile der Eintrittsflächen sind nicht auf die in diesen Fig. 5-9 gezeigten quadratischen bzw. rechteckigen Formen beschränkt, sondern können auch die Form anderer Polygone haben, beispielsweise die Form eines Pentagons oder eines Oktogons. Auch bei der Ausgestaltung in Form anderer Polygone als Vierecke ist es eine bevorzugte Massnahme, dass die Polygone mit abgerundeten Ecken zwischen aneinander angrenzenden Kanten 225 ausgestaltet sind.

Bei der Ausgestaltung der Profile der Eintrittsflächen 221 in Form von Polygonen ist mit der Profilhöhe D1 die maximale Höhe des Profils in axialer Richtung A gemeint und mit der Profilbreite D2 die maximale Erstreckung des Profils in radialer Richtung. Besonders bevorzugt ist das Profil als ein Polygon mit der Profilhöhe D1 derart ausgestaltet, dass die Querschnittsfläche der Eintrittsfläche grösser ist als eine Kreisfläche, deren Durchmesser die Profilhöhe D1 ist.

Das Pumpengehäuse 2 ist vorzugsweise zweiteilig ausgestaltet, nämlich mit dem separaten Bodenteil 3 und dem Deckelteil 4, die dermassen dichtend miteinander verbunden werden, dass sie gemeinsam das Pumpengehäuse 2 mit dem darin angeordneten Rotor 10 bilden. Im Folgenden werden anhand der Fig. 10 und Fig. 11 ein zweites und ein drittes Ausführungsbeispiel einer erfindungsgemässen Pumpeneinheit 1 beschrieben, die sich durch die Trennung zwischen dem Bodenteil 3 und dem Deckelteil 4 unterscheiden. Fig. 10 zeigt das zweite Ausführungsbeispiel in einer Schnittdarstellung entlang der axialen Richtung A. Bei dem zweiten Ausführungsbeispiel ist der Auslass 22 im Bodenteil 3 angeordnet, während der Einlass 21 im Deckelteil 4 angeordnet ist. Fig. 11 zeigt das dritte Ausführungsbeispiel in einer Schnittdarstellung entlang der axialen Richtung A. Bei dem dritten Ausführungsbeispiel sind sowohl der Einlass 21 als auch der Auslass 22 im Deckelteil 4 angeordnet.

Bei der folgenden Beschreibung des zweiten und des dritten Ausführungsbeispiels wird nur auf die Trennung zwischen dem Bodenteil 3 und dem Deckelteil 4 näher eingegangen. Gleiche Teile oder von der Funktion her gleichwertige Teile des zweiten und dritten Ausführungsbeispiels sind mit den gleichen Bezugszeichen bezeichnet wie bei dem ersten Ausführungsbeispiel. Insbesondere haben die Bezugszeichen die gleiche Bedeutung wie sie bereits im Zusammenhang mit dem ersten Ausführungsbeispiel erläutert sind. Es versteht sich, dass alle vorangehenden Erläuterungen des ersten Ausführungsbeispiels insbesondere diejenigen bezüglich des Auslasses 22 und seiner Eintrittsfläche 221 in gleicher Weise oder in sinngemäss gleicher Weise auch für das zweite und das dritte Ausführungsbeispiel gelten.

Bei dem zweiten Ausführungsbeispiel, das in Fig. 10 dargestellt ist, ist der Auslass 22 und insbesondere die Eintrittsfläche 221 des Auslasses 22 im Bodenteil 3 angeordnet, während der Einlass 21 im Deckelteil 4 angeordnet ist. Das Bodenteil 3 ist dichtend mit dem Deckelteil 4 verbunden. Das Deckelteil 4 ist beispielsweise über einen Presssitz mit dem Bodenteil 3 verbunden. Alternativ ist es natürlich auch möglich, das Bodenteil 3 mit dem Deckelteil 4 zu verschweissen, um so eine dichtende Verbindung zwischen dem Bodenteil 3 und dem Deckelteil 4 zu realisieren.

Zwischen dem Bodenteil 3 und dem Deckelteil 4 ist ein Dichtungselement 90 vorgesehen, beispielsweise ein O-Ring oder eine Flachdichtung, um eine Leckage des Fluids in die Umgebung zu vermeiden. Für die Betriebssicherheit der Pumpeneinheit 1 ist eine zuverlässig dichtende Verbindung zwischen dem Bodenteil 3 und dem Deckelteil 4 vorteilhaft, sodass Leckagen des Fluids aus dem Inneren des Pumpengehäuses 2 zwischen dem Bodenteil 3 und dem Deckelteil 4 hindurch in den Aussenraum ausserhalb des Pumpengehäuses 2 zuverlässig verhindert werden können. Für manche Anwendungen soll diese dichtende Verbindung auch bei hohen Temperaturen von beispielsweise bis zu 220°C und/oder bei hohen Drücken und/oder für chemisch sehr aggressive Fluide, wie beispielsweise Schwefelsäure gewährleistet sein.

Das Dichtungselement 90 ist vorzugsweise als radiales Dichtungselement 90 ausgestaltet. Das bedeutet beispielsweise bei einer Ausgestaltung als O-Ring, dass das Dichtungselement 90 bezüglich der radialen Richtung zwischen dem Deckelteil 4 und dem Bodenteil 3 angeordnet ist. Bei einer Ausgestaltung als axiales Dichtelement ist das Dichtelement bezüglich der axialen Richtung A zwischen dem Bodenteil und dem Deckelteil 4 angeordnet. Allgemeiner ausgedrückt ist das radiale Dichtungselement 90 in einer gekrümmten Fläche angeordnet, während ein axiales Dichtelement in einer planaren, das heisst nicht gekrümmten, Fläche angeordnet ist.

Für das Dichtungselement 90 werden Elastomere bevorzugt, insbesondere auch deshalb, weil Elastomere sehr gute Rückstellkräfte aufweisen. In der Halbleiterindustrie, beispielsweise, wo extrem hohe Anforderungen an die Reinheit gestellt werden, ist es auch üblich, Perfluorelastomere (Perfluorkautschuk, FFPM) für das Dichtungselement 90 zu verwenden. FFPM wird insbesondere dort eingesetzt, wo eine sehr gute thermische und/oder chemische Beständigkeit notwendig ist.

Die Ausgestaltung, bei welcher der Auslass 22 im Bodenteil 3 angeordnet ist, hat den Vorteil, dass die Eintrittsfläche 221 des Auslasses 22 bezüglich der axialen Richtung A sehr nahe bzw. mit einem grossen Überlapp mit den Flügeln 103 des Rotors 10 angeordnet werden kann, also darstellungsgemäss (Fig. 10) sehr weit unten, weil unterhalb des Auslasses 22 keine Dichtungen mehr notwendig sind.

Bei dem in Fig. 11 dargestellten dritten Ausführungsbeispiel der erfindungsgemässen Pumpeneinheit 1 sind sowohl der Einlass 21 als auch der Auslass 22 am Deckelteil 4 angeordnet.

Bezüglich der Verbindung zwischen dem Deckelteil 4 und dem Bodenteil 3 sowie dem dazwischen angeordneten Dichtungselement 90 gelten für das dritte Ausführungsbeispiel die Erläuterungen zum zweiten Ausführungsbeispiel in analoger Weise. Der Unterschied zwischen dem zweiten und dem dritten Ausführungsbeispiel ist es, dass beim dritten Ausführungsbeispiel die Trennung zwischen dem Bodenteil 3 und dem Deckelteil 4 darstellungsgemäss (Fig. 11) und bezüglich der axialen Richtung A unterhalb des Auslasses 22 bzw. unterhalb der Eintrittsfläche 221 in den Auslass 22 angeordnet ist, während bei dem zweiten Ausführungsbeispiel (Fig. 10) diese Trennung oberhalb des Auslasses 22 bzw. der Eintrittsfläche 221 in den Auslass 22 angeordnet ist.

Die Ausgestaltung des dritten Ausführungsbeispiels, bei welchem sowohl der Einlass 21 als auch der Auslass 22 des Pumpengehäuses 2 am Deckelteil 4 angeordnet sind, hat den Vorteil, dass der Einfluss der am Einlass 21 und am Auslass 22 angeschlossenen Verrohrungssysteme im Hinblick auf potentielle Leckagen erheblich reduziert wird, wodurch sich die Betriebssicherheit steigern lässt. Da der Einlass 21 und der Auslass 22 am Deckelteil 4 angeordnet sind, führen die mechanischen Kräfte, welche die dort angeschlossenen Leitungen bzw. Rohre auf das Pumpengehäuse 2 ausüben, nicht mehr zu Relativbewegungen zwischen dem Deckelteil 4 und dem Bodenteil 3, wie das der Fall sein könnte, wenn beispielsweise der Einlass 21 am Deckelteil 4 und der Auslass 22 am Bodenteil 3 angeordnet sind. Mechanische Momente, wie Kipp-, Scher- oder Torsionsmomente, welche die Abdichtung zwischen dem Deckelteil 4 und dem Bodenteil 3 insbesondere mechanisch belasten, werden durch die Ausgestaltung gemäss des dritten Ausführungsbeispiels reduziert.

Ferner ist es bei dieser Ausgestaltung vorteilhafterweise möglich, das Deckelteil 4 aus einem mechanisch sehr stabilen Material, beispielsweise einem metallischen Werkstoff zu fertigen, das sehr gut grosse Kräfte aufnehmen kann, während das Bodenteil 4 und insbesondere der Becher 31 aus einem Kunststoff gefertigt werden kann, was vorteilhaft im Hinblick auf die magnetische Wechselwirkung mit dem Stator 100 ist.

Allerdings bedingt die Ausgestaltung des dritten Ausführungsbeispiels, bei welcher die Trennung zwischen dem Bodenteil 3 und dem Deckelteil 4 darstellungsgemäss (Fig. 11) unterhalb der Eintrittsfläche 221 in den Auslass 22 angeordnet ist, dass die Eintrittsfläche 221 bezüglich der axialen Richtung A darstellungsgemäss (Fig. 11) etwas weiter nach oben geschoben und damit weiter weg von den Flügeln 103 angeordnet werden muss, weil darstellungsgemäss unterhalb der Eintrittsfläche 221 noch die Trennung zwischen dem Bodenteil 3 und dem Deckelteil 4 mit dem Dichtungselement 90 angeordnet werden muss.

In dieser Hinsicht ist die erfindungsgemässe Ausgestaltung der Eintrittsfläche 221 besonders vorteilhaft, weil es - beispielsweise im Vergleich mit einem kreisförmigen Profil der Eintrittsfläche (Fig. 3) - bei gleich grosser Querschnittsfläche des Profils die erfindungsgemässe Ausgestaltung des Profils der Eintrittsfläche 221 ermöglicht, die Eintrittsfläche 221, genauer gesagt, ihre Mittellinie oder ihren Schwerpunkt ES, bezüglich der axialen Richtung A näher an den Flügeln 103 anzuordnen.

Für die Erfindung ist es keinesfalls notwendig, sondern höchstens bevorzugt, dass das Profil der Eintrittsfläche 221 eine Symmetrieachse aufweist. Es ist beispielsweise auch möglich, das Profil der Eintrittsfläche 221 optimal an den im Pumpenraum 23 vorhandenen Platz anzupassen. Fig. 12 zeigt ein viertes Ausführungsbeispiel einer erfindungsgemässen Pumpeneinheit 1 in einer Schnittdarstellung entlang der axialen Richtung A. Im Folgenden wird nur auf die Unterschiede zu den vorangehend beschriebenen Ausführungsbeispielen näher eingegangen. Ansonsten gelten die vorangehenden Erläuterungen auch für das vierte Ausführungsbeispiel.

Bei dem vierten Ausführungsbeispiel umfasst der Deckelteil 4 einen zylindrischen Bereich 41, welcher mit dem Bodenteil 3 verbunden ist, sowie einen sich daran in axialer Richtung A anschliessenden konischen Bereich 42, der sich in Richtung des Einlasses 21 verjüngt. Die Eintrittsfläche 221 in den Auslass 22 ist im Übergang vom zylindrischen Bereich 41 in den konischen Bereich 42 an der Wandung des Pumpengehäuses 2 angeordnet. Das Profil der Eintrittsfläche 221 hat die Form eines unregelmässigen Sechsecks, wobei eine Kante 225 des Sechsecks von dem zylindrischen Bereich 41 gebildet wird, und eine Kante 225 von dem konischen Bereich 42. Es ist gut zu erkennen, dass sich das Profil der Eintrittsfläche 221 in sehr guter und einfacher Weise an die Geometrie des Pumpenraums 23 anpassen lässt. Ferner ist zu erkennen, dass dieses Profil eine grössere Querschnittsfläche hat als jedes kreisförmige Profil, das in dem gleichen Raum angeordnet wird.

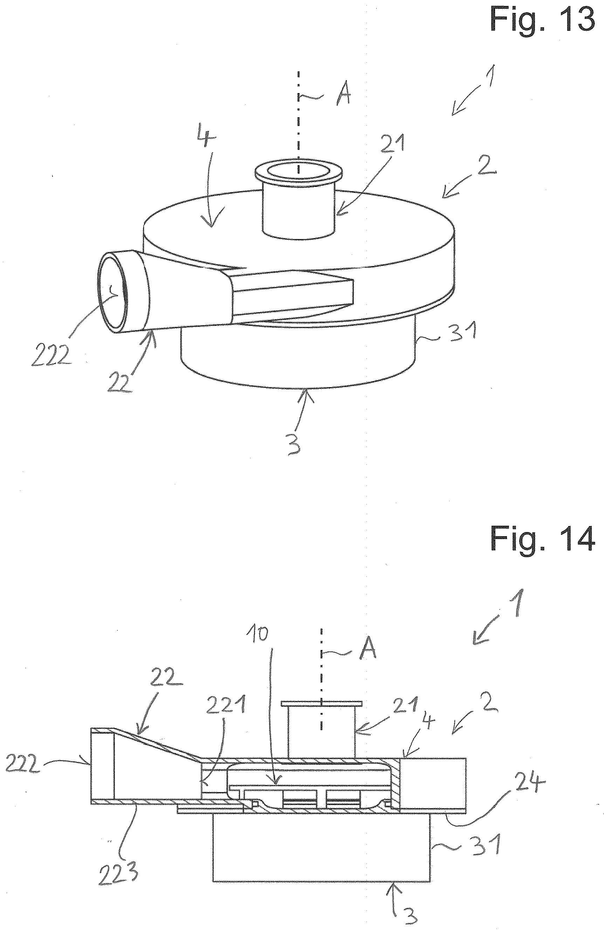

Fig. 13 zeigt eine perspektivische Darstellung eines fünften Ausführungsbeispiels einer erfindungsgemässen Pumpeneinheit 1. Zum besseren Verständnis zeigt Fig. 14 das fünfte Ausführungsbeispiel noch in einer Schnittdarstellung, wobei der Schnitt in axialer Richtung A erfolgt. Im Folgenden wird nur auf die Unterschiede zu den vorangehend beschriebenen Ausführungsbeispielen näher eingegangen. Ansonsten gelten die vorangehenden Erläuterungen auch für das fünfte Ausführungsbeispiel.

Das fünfte Ausführungsbeispiel bezieht sich insbesondere auf die Ausgestaltung des Auslasses 22. Es versteht sich, dass die nachfolgend erläuterte Ausgestaltung des Auslasses 22 in sinngemäss gleicher Weise auch für die vorangehend beschriebenen Ausführungsbeispiele verwendet werden kann.

Der Auslass 22 hat die Eintrittsfläche 221, durch welche das Fluid in den Auslass 22 einströmen kann, und die Austrittsfläche 222, durch welche das Fluid aus dem Auslass 22 herausströmt. Die Eintrittsfläche 221 ist erfindungsgemäss ausgestaltet, beispielsweise mit einem Profil, das im Wesentlichen rechteckförmig oder quadratisch ausgestaltet ist, wobei die Ecken abgerundet sein können, so wie dies beispielsweise in Fig. 11 dargestellt ist. Die Austrittsfläche 222 ist mit einem kreisförmigen Profil ausgestaltet. Vorzugsweise ist die Querschnittsfläche der Austrittsfläche 222 grösser als die Querschnittsfläche der Eintrittsfläche 221. Ferner ist es bevorzugt, dass sich der Auslass 22 in Strömungsrichtung konisch erweitert, sodass der Auslass 22 als Diffusor ausgestaltet ist. Der freie Strömungsquerschnitt des Auslasses 22 erweitert sich somit in Strömungsrichtung gesehen konisch und ändert sich von einem im Wesentlichen rechteckigen oder quadratischen Querschnitt zu einem kreisförmigen Querschnitt. Im Bereich der Austrittsfläche 222 ist der Auslass 22 rund ausgestaltet, sodass er in einfacher Weise mit einer Leitung oder mit einem Schlauch verbunden werden kann.

Die konische Erweiterung des Auslasses 22 ist vorzugsweise asymmetrisch ausgestaltet, derart, dass der Auslass 22 einen flachen Boden 223 aufweist, der zum Anliegen an dem ersten axialen Ende 110 des Stators 100 ausgestaltet ist. Dazu erstreckt sich der flache Boden 223 senkrecht zur axialen Richtung A, also im Wesentlichen parallel zu dem flanschartigen Vorsprung 24 des Pumpengehäuses 2.

Das hat den Vorteil, dass der Auslass 22 möglichst nahe am Stator 100 angeordnet werden kann, in sinngemäss gleicher Weise wie dies in Fig. 1 für die aus dem Stand der Technik bekannte Pumpeneinheit 1' dargestellt ist.

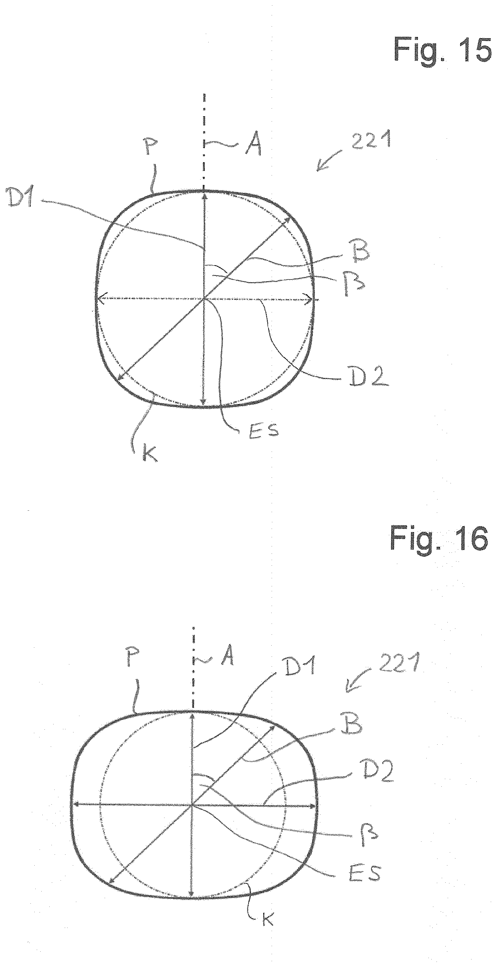

Im Folgenden werden anhand der Fig. 15 und Fig. 16 noch zwei Varianten für das Profil der Eintrittsfläche 221 des Auslasses 22 erläutert, die für alle beschriebenen Ausführungsbeispiele der erfindungsgemässen Pumpeneinheit 1 verwendet werden können. Bei den vorangehend beschriebenen Ausführungsbeispielen der Pumpeneinheit 1 ist das Profil der Eintrittsfläche 221 jeweils mit mehreren geradlinigen Kanten 225 ausgestaltet, beispielsweise ist die Eintrittsfläche 221 mit einem im Wesentlichen rechteckigen oder quadratischen Profil und optional mit abgerundeten Ecken zwischen den geradlinigen Kanten 225 ausgestaltet. Es sind aber auch solche Ausgestaltungen möglich, bei denen das Profil stärker abgerundet oder auch ganz ohne geradlinige Kanten ausgestaltet ist.

In Fig. 15 ist eine erste Variante für das Profil der Eintrittsfläche 221 dargestellt. Das Profil ist mit dem Bezugszeichen P bezeichnet. Bei dieser ersten Variante ist die Profilhöhe D1 gleich gross wie die Profilbreite D2. Im Vergleich zu der beispielsweise in Fig. 5 dargestellten, im Wesentlichen quadratischen Ausgestaltung des Profils ist das Profil P gemäss Fig. 15 deutlich stärker abgerundet ausgestaltet.

Zum Vergleich ist in Fig. 15 noch eine Kreisfläche K eingezeichnet, deren Durchmesser gleich gross ist wie die Profilhöhe D1 bzw. die Profilbreite D2. Es ist deutlich zu erkennen, dass die Querschnittsfläche des Profils P grösser ist als die Fläche des Kreises K.

Durch das von einer Kreisfläche abweichende Profil P der Eintrittsfläche 221 ist es möglich, das Profil P der Eintrittsfläche 221 des Auslasses 22 mit einer grösseren Querschnittsfläche auszugestalten als bei der kreisförmigen Ausgestaltung des Profils (siehe z.B. Fig. 3), ohne dass dafür die geometrischen Abmessungen des Pumpengehäuses 2 geändert werden müssen. Eine grössere Querschnittsfläche des Profils P der Eintrittsfläche 221 des Auslasses 22 hat den Vorteil, dass der Druckabfall über den Auslass 22, der im Wesentlichen durch diese Querschnittsfläche bestimmt wird, reduziert werden kann, was im Hinblick auf die Effizienz der Pumpeneinheit 1 vorteilhaft ist. Durch den reduzierten Druckabfall über den Auslass 22 erhöht sich der Wirkungsgrad der Zentrifugalpumpe 200.

In Fig. 16 ist eine zweite Variante für das Profil P der Eintrittsfläche 221 dargestellt. Bei der in Fig. 16 dargestellten zweiten Variante ist die Profilbreite D2 grösser als die Profilhöhe D1. Durch diese Ausgestaltung des Profils P verschiebt sich der Mittelpunkt des Profils P, oder im allgemeineren Fall eines nicht symmetrischen Profils der Schwerpunkt ES des Profils, bezüglich der axialen Richtung A darstellungsgemäss nach unten im Vergleich zu einem kreisförmigen Profil gleicher Querschnittsfläche. Bei dieser Ausgestaltung, bei welcher die Profilbreite D2 grösser ist als die Profilhöhe D1, liegt der Schwerpunkt ES des Profils P näher an der Ebene, welche die Mittellinien ME aller Flügel 103 enthält.

Es sind verschiedene Ausgestaltungen des Profils P möglich, bei denen die Profilbreite D2 grösser ist als die Profilhöhe D1. In Fig. 16 ist beispielsweise eine Ausgestaltung als stark ausgerundetes Rechteck gezeigt. Die Ausrundung des Rechtecks kann dabei so stark sein, dass das Profil keine im strengen Sinne geradlinigen Kanten mehr umfasst. Weiterhin ist es möglich, das Profil ellipsenförmig auszugestalten. Im Allgemeinen sind bei der zweiten Variante solche Ausgestaltungen bevorzugt, bei denen es mindestens eine zweite Richtung B gibt, in welcher das Profil P eine maximale Erstreckung aufweist, die grösser ist als die Profilhöhe D1, also die maximale Erstreckung des Profils in axialer Richtung A, wobei diese zweite Richtung B mit der axialen Richtung A einen von null verschiedenen Winkel β einschliesst. Bei der Darstellung in Fig. 16 schliesst die zweite Richtung B mit beispielhaftem Charakter den Winkel β = 45° mit der axialen Richtung A ein.

Durch die Erfindung wird ferner die Zentrifugalpumpe 200 zum Fördern eines Fluids mit einer Pumpeneinheit 1 vorgeschlagen, wobei die Pumpeneinheit 1 erfindungsgemäss ausgestaltet ist. Fig. 17 zeigt in einer schematischen Schnittdarstellung ein Ausführungsbeispiel einer erfindungsgemässen Zentrifugalpumpe 200. Die Zentrifugalpumpe 200 umfasst den Stator 100, der sich in der axialen Richtung A von einem ersten axialen Ende 110 bis zu einem zweiten axialen Ende 120 erstreckt, wobei an dem ersten axialen Ende 110 eine becherförmige Ausnehmung (in Fig. 17 nicht dargestellt) vorgesehen ist, in welche der zylindrischen Becher 31 der Pumpeneinheit 1 einsetzbar ist. Der Stator 100 bildet mit dem Rotor 10 einen elektromagnetischen Drehantrieb zum Rotieren des Rotors 10 um die axiale Richtung A, wobei der Stator 100 als Lager- und Antriebsstator ausgestaltet ist, mit welchem der Rotor 10 berührungslos magnetisch antreibbar und berührungslos magnetisch bezüglich des Stators 100 lagerbar ist, wobei der Rotor 10 bezüglich der axialen Richtung A passiv magnetisch stabilisiert ist, und in einer zur axialen Richtung A senkrechten radialen Ebene E aktiv magnetisch gelagert ist.

Der Stator 100 umfasst ein Statorgehäuse, das in Fig. 17 aus Gründen der besseren Übersicht nicht dargestellt ist. Der Stator 100 kann aber beispielsweise in analoger Weise ausgestaltet sein, wie der in Fig. 1 dargestellte Stator 100' mit dem Statorgehäuse 130`, wobei in dem Statorgehäuse 130' die Ausnehmung 121' vorgesehen ist, in welche der zylindrische Becher 31 des Bodenteils 3 des Pumpengehäuses 1 eingesetzt wird.

Besonders bevorzugt ist der elektromagnetische Drehantrieb mit dem Rotor 10 und dem Stator 100 als Tempelmotor ausgestaltet, wobei der Stator 100 eine Mehrzahl von Spulenkernen 125 aufweist, von denen jeder einen Längsschenkel 126 umfasst, welcher sich von einem ersten Ende in axialer Richtung A bis zu einem zweiten Ende erstreckt, sowie einen Querschenkel 127, welcher an dem zweiten Ende des Längsschenkels 126 und in der radialen Ebene E angeordnet ist. Der Querschenkel 127 erstreckt sich von dem Längsschenkel 126 in radialer Richtung nach innen auf den Rotor 10 zu.

Alle ersten Enden der Längsschenkel 126 - also die darstellungsgemäss unteren Enden - sind durch einen Rückschluss 122 zum Führen des magnetischen Flusses miteinander verbunden.

Die Spulenkerne 125 sind bezüglich der Umfangsrichtung um den Rotor 10 herum angeordnet sind, sodass der Rotor 10 zwischen den Querschenkeln 127 der Spulenkerne 125 angeordnet ist. An jedem Längsschenkel 126 ist mindestens eine konzentrierte Wicklung 160 vorgesehen, welche den jeweiligen Längsschenkel 126 umgibt.

Mit den konzentrierten Wicklungen 160 werden die für den magnetischen Antrieb und die magnetische Lagerung des Rotors 10 notwendigen elektromagnetischen Felder erzeugt. Mit diesen konzentrierten Wicklungen 160 werden im Betriebszustand also diejenigen elektromagnetischen Felder erzeugt, mit welchen in an sich bekannter Weise ein Drehmoment auf den Rotor 10 bewirkt wird, und mit welchen eine beliebig einstellbare Querkraft in radialer Richtung auf den Rotor 10 ausübbar ist, sodass die radiale Position des Rotors 10, also seine Position in der zur axialen Richtung A senkrechten radialen Ebene E, aktiv steuerbar bzw. regelbar ist. Bezüglich dreier weiterer Freiheitsgrade, nämlich seiner Position in axialer Richtung A und Verkippungen bezüglich der zur Solldrehachse senkrechten radialen Ebene E (zwei Freiheitsgrade), ist der Rotor 10 passiv magnetisch, das heisst nicht ansteuerbar, durch Reluktanzkräfte gelagert bzw. stabilisiert.

## Patentansprüche

1. Pumpeneinheit für eine Zentrifugalpumpe, welche die Pumpeneinheit und einen Stator (100) umfasst, der sich in einer axialen Richtung (A) von einem ersten axialen Ende (110) bis zu einem zweiten axialen Ende (120) erstreckt, wobei an dem ersten axialen Ende (110) eine becherförmige Ausnehmung vorgesehen ist, in welche die Pumpeneinheit (1) einsetzbar ist, wobei die Pumpeneinheit (1) ein Pumpengehäuse (2) mit einem Einlass (21) und mit einem Auslass (22) für ein zu förderndes Fluid aufweist, sowie einen in dem Pumpengehäuse (2) angeordneten Rotor (10) mit einer Mehrzahl von Flügeln (103) zum Fördern des Fluids, wobei das Pumpengehäuse (2) einen Pumpenraum (23) begrenzt, wobei der Rotor (10) um die axiale Richtung (A) rotierbar ist, wobei die Pumpeneinheit (1) für eine berührungslos magnetische Lagerung des Rotors (10) und für einen berührungslos magnetischen Antrieb des Rotors (10) durch den Stator (100) ausgestaltet ist, wobei das Pumpengehäuse (2) ein Deckelteil (4) und ein Bodenteil (3) aufweist, wobei das Bodenteil (3) einen zylindrischen Becher (31) zur Aufnahme des Rotors (10) aufweist, welcher Becher in die becherförmige Ausnehmung des Stators (100) einsetzbar ist, und wobei der Auslass (22) eine Eintrittsfläche (221) aufweist, durch welche das Fluid aus dem Pumpenraum (23) in den Auslass (22) strömen kann, **dadurch gekennzeichnet, dass** die Eintrittsfläche (221) des Auslasses (22) ein Profil aufweist, welches verschieden von einer Kreisfläche ist.

2. Pumpeneinheit nach Anspruch 1, wobei das Profil (P) der Eintrittsfläche (221) eine Profilhöhe (D1) in axialer Richtung aufweist, wobei es mindestens eine zweite Richtung (B) gibt, die mit der axialen Richtung (A) einen von null verschiedenen Winkel (β) einschliesst, und wobei die maximale Erstreckung des Profils in der zweiten Richtung (B) grösser ist als die Profilhöhe (D1).

3. Pumpeneinheit nach Anspruch 2, wobei der Winkel (β) zwischen der zweiten Richtung (B) und der axialen Richtung (A) 90° beträgt.

4. Pumpeneinheit nach einem der vorangehenden Ansprüche, wobei das Profil der Eintrittsfläche (221) des Auslasses (22) mindestens eine und vorzugsweise mehrere geradlinigen Kanten (225) aufweist.

5. Pumpeneinheit nach einem der vorangehenden Ansprüche, wobei das Profil der Eintrittsfläche (221) genau vier geradlinige (225) Kanten aufweist und vorzugsweise im Wesentlichen rechteckförmig ausgestaltet ist.

6. Pumpeneinheit nach einem der vorangehenden Ansprüche, wobei zwei benachbarte geradlinige Kanten (225) des Profils durch eine Abrundung verbunden sind.

7. Pumpeneinheit nach einem der vorangehenden Ansprüche, wobei jeder Flügel (103) eine Austrittskante (103a) aufweist, sowie eine zur axialen Richtung (A) senkrechte Mittellinie (ME), welche den jeweiligen Flügel (103) an der Austrittskante (103a) bezüglich der axialen Richtung (A) in zwei gleich hohe Abschnitte teilt, und wobei die Mittellinien (ME) aller Flügel (103) in einer Ebene liegen.

8. Pumpeneinheit nach Anspruch 7, wobei das Profil der Eintrittsfläche (221) einen Schwerpunkt (ES) aufweist, wobei jeder Flügel (103) an der Austrittskante (103a) eine Höhe (H) in axialer Richtung (A) hat, und wobei der Schwerpunkt (ES) des Profils der Eintrittsfläche (221) einen Abstand von der Ebene aufweist, in welcher die Mittellinien (ME) der Flügel (103) liegen, der höchsten das 1.5-fache der Höhe (H) der Flügel (103) an der Austrittskante (103a) beträgt.

9. Pumpeneinheit nach Anspruch 8, wobei der Schwerpunkt (ES) des Profils der Eintrittsfläche (221) einen Abstand von der Ebene aufweist, in welcher die Mittellinien (ME) der Flügel (103) liegen, der höchstens das 1.1-fache der Höhe (H) der Flügel (103) an der Austrittskante (103a) beträgt.

10. Pumpeneinheit nach einem der vorangehenden Ansprüche, wobei der Auslass (22) eine Austrittsfläche (222) aufweist, durch welche das Fluid aus dem Auslass (22) herausströmen kann, wobei die Austrittsfläche (222) als Kreisfläche ausgestaltet ist.

11. Pumpeneinheit nach Anspruch 10, wobei die Austrittsfläche (222) eine grössere Querschnittsfläche aufweist als die Eintrittsfläche (221).

12. Pumpeneinheit nach einem der Ansprüche 10-11 wobei der Auslass (22) in Strömungsrichtung gesehen sich konisch erweiternd ausgestaltet ist.

13. Pumpeneinheit nach einem der vorangehenden Ansprüche, wobei der Auslass (22) einen flachen Boden (223) aufweist, der zum Anliegen an dem ersten axialen Ende des Stators ausgestaltet ist.

14. Zentrifugalpumpe zum Fördern eines Fluids mit einer Pumpeneinheit, die nach einem der vorangehenden Ansprüche ausgestaltet ist, und mit einem Stator (100), der sich in einer axialen Richtung (A) von einem ersten axialen Ende (110) bis zu einem zweiten axialen Ende (120) erstreckt, wobei an dem ersten axialen Ende (110) eine becherförmige Ausnehmung vorgesehen ist, in welche der zylindrischen Becher (31) der Pumpeneinheit (1) einsetzbar ist, wobei der Stator (100) mit dem Rotor (10) einen elektromagnetischen Drehantrieb zum Rotieren des Rotors (10) um die axiale Richtung (A) bildet, wobei der Stator (100) als Lager- und Antriebsstator ausgestaltet ist, mit welchem der Rotor (10) berührungslos magnetisch antreibbar und berührungslos magnetisch bezüglich des Stators (100) lagerbar ist, wobei der Rotor (10) bezüglich der axialen Richtung (A) passiv magnetisch stabilisiert ist, und in einer zur axialen Richtung (A) senkrechten radialen Ebene aktiv magnetisch gelagert ist.

15. Zentrifugalpumpe nach Anspruch 14, bei welchem der elektromagnetische Drehantrieb als Tempelmotor ausgestaltet ist, wobei der Stator (100) eine Mehrzahl von Spulenkernen (125) aufweist, von denen jeder einen Längsschenkel (126) umfasst, welcher sich von einem ersten Ende in axialer Richtung (A) bis zu einem zweiten Ende erstreckt, sowie einen Querschenkel (127), welcher an dem zweiten Ende des Längsschenkels (126) und in der radialen Ebene angeordnet ist, und welcher sich von dem Längsschenkel (126) in radialer Richtung erstreckt, wobei die Spulenkerne (125) bezüglich der Umfangsrichtung um den Rotor (10) herum angeordnet sind, sodass der Rotor (10) zwischen den Querschenkeln (127) der Spulenkerne (125) angeordnet ist, und wobei an jedem Längsschenkel (125) mindestens eine konzentrierte Wicklung (160) vorgesehen ist, welche den jeweiligen Längsschenkel (126) umgibt.
